# EUROPÄISCHE PATENTSCHRIFT

(11) **EP 1 220 627 B1**
(45) Veröffentlichungstag und Bekanntmachung des Hinweises auf die Patenterteilung: **02.03.2005**
(21) Anmeldenummer: 01951403.3
(22) Anmeldetag: 20.06.2001
(51) Int. Cl.: A47C 20/02, A61G 7/065, A47C 27/08

(54) **AUFBLASBARES KISSEN**
INFLATABLE CUSHION
COUSSIN GONFLABLE

(30) Priorität: 20.06.2000 DE 20010921 U
(43) Veröffentlichungstag der Anmeldung: 10.07.2002
(73) Patentinhaber: Bücken, Uwe, 50259 Pulheim (DE); Pütz, Günther, 50933 Köln (DE)
(72) Erfinder: Bücken, Uwe, 50259 Pulheim (DE); Pütz, Günther, 50933 Köln (DE)
(74) Vertreter: Kreutzer, Ulrich, Dipl.-Phys.
(86) Internationale Anmeldenummer: PCT/DE2001/002252
(87) Internationale Veröffentlichungsnummer: WO 2001/097658

(56) Entgegenhaltungen:
- DE-U- 29 809 439
- US-A- 5 418 991
- US-A- 6 065 166

## Beschreibung

Die vorliegende Erfindung betrifft ein aufblasbares Kissen sowie dessen Verwendung zur Behandlung orthopädischer Erkrankungen.

Aufblasbare Kissen sind weit verbreitet. Sie haben im wesentlich den Vorteil, in nicht aufgeblasenem Zustand in platzsparender Weise leicht transportabel zu sein und durch die Luftpolsterung eine möglichst gleichmäßige Druckentlastung für die entsprechenden Körperpartien bereitzustellen.

So ist beispielsweise in der DE-OS 39 12 709 A1 ein medizinisches Sitzkissen offenbart, das aufgrund des elastischen, druckverteilenden Materials die durch Druckempfindlichkeit eines Teils der Skelettmuskulatur hervorgerufenen Schmerzen lindert.
Zwar weist dieses Sitzkissen anatomisch bedingte Aussparungen für bestimmte Körperregionen auf, jedoch ist die Oberfläche des Sitzkissens flach gehalten, so dass nur durch Zusammenwirken von Körpergewicht und Kompressionsmodul des elastomeren Materials die erforderliche Druckentlastung erreicht wird.

Ferner ist aus der DE 33 00 851 A1 ein Stützpolster zur Verwendung auf Autositzen bekannt, dessen auf die Wirbelsäule wirkender Druckbereich keine gleichbleibende Verformung und Elastizität aufweist. Vielmehr weist dieses Stützpolster einen starren Rahmen auf, wodurch u.a. eine zu starke Wölbung der Polsterhinterseite verhindert werden soll.
Was die physiotherapeutischen Eigenschaften betrifft, so steht bei diesem Kissen hierbei die Massagefunktion im Vordergrund, speziell im Hinblick während längerer Autofahrten nicht jedoch die Druckentlastung für die Wirbelsäule, was insbesondere bei orthopädischen Erkrankungen der Halswirbelsäule von vorrangiger Bedeutung ist.

Aufgabe der vorliegenden Erfindung ist die Bereitstellung eines aufblasbaren Kissens, das sich aufgrund seiner Form und seiner druckentlastenden Eigenschaften besonders zur Behandlung von Erkrankungen der Halswirbelsäule, Schulter und/oder des Kniegelenks eignet.
Dabei ist es wichtig, das sich ein solches Kissen den anatomischen Eigenschaften der Halswirbelsäule, Schulter und/oder des Kniegelenks anpasst ohne die orthopädisch wichtige, stützende Funktion zu beeinträchtigen.

Diese Aufgabe wird erfindungsgemäß gelöst durch ein aufblasbares Kissen aus mindestens zwei miteinander verbundenen Folien, von denen die eine Folie die Unterseite des Kissens bildet und die andere Folie elastomere Eigenschaften aufweist und die Oberseite des Kissens bildet, im aufgeblasenen Zustand mit einer Breite (B) zwischen 50 und 80 cm, einer Länge (L) zwischen 10 und 30 cm, und einer Höhe (H) von maximal 10 cm, umfassend eine im wesentlichen ebene Unterseite (1), eine im Querschnitt der Längsrichtung betrachtet an beiden Randbereichen je eine konvex gewölbte Oberseite (2) und (2a) und mindestens eine, zwischen den Randbereichen in Richtung der Unterseite konkav verlaufende Mulde (3) der Oberseite.

Eine wesentliche Eigenschaft dieses erfindungsgemäßen Kissens ist, dass die konkav verlaufende Ausbuchtung eine Vorformung für die entsprechenden Körperpartien bildet, und die darüber hinaus erforderliche anatomische Anpassung durch die elastomeren Eigenschaften der die Oberseite des Kissens bildenden Folie gewährleistet ist.
Hierdurch werden die sonst unvermeidlichen Druckpunkte im Hinterkopf und Schultemackenbereich bzw. im Bereich der ischiocrualen Muskulatur und Wade vermieden.
Neben der für den Anwender zu beobachtenden Entspannung der Trapeziusmuskulatur und Halswirbelsäulenmuskulatur bzw. ischiocrualen Muskulatur und Unterschenkelmuskulatur werden Stauchungen der Arterien und Venen, Stoffwechselstau, Lymphstau, Sensibilitätsstörungen oder Nervenreizungen vermieden und eine optimale Lagerung für die entsprechenden Gelenkkapseln erreicht.
Eine optimale Anpassung an die anatomischen Gegebenheiten des erfindungsgemäßen Kissens unter Beibehaltung ausreichender Stützungseigenschaften kann durch einen einfachen Test ermittelt werden, bei dem die notwendige Druckentlastung gegeben ist, wenn eine zylindrische Rolle mit einem Durchmesser von 12 cm, einer Länge von 15 cm und einem Gewicht von 8 kg in die Mulde (3) des aufgeblasenen Kissens gelegt wird und sich die Mulde (3) um mindestens 1 cm senkt. Eine ausreichende Stützfunktion des Kissens im aufgeblasenen Zustand kann dann festgestellt werden, wenn auf die zuvor beschriebene Rolle eine seitliche Kraft von 10 N in axialer Richtung ausgeübt wird ohne dass sich die Rolle über das Kissen aus der Mulde heraus bewegt. Dies ist in einfacher Weise realisierbar, wenn ein Bügel an beiden Enden der Rolle mit deren Mittelachse drehbar gelagert angebracht ist und die Mitte des Bügels mittels eines über eine Umlenkrolle verlaufenden Drahts mit einem frei hängenden Gewicht von 1 kg verbunden ist.
In einer weiteren erfindungsgemäßen Ausführungsform weist das Kissen zusätzlich mindestens einen Mittelsteg (4) auf, der in Längsrichtung des Kissens verläuft und mit der Oberseite und der Unterseite verbunden ist.

So kann erreicht werden, dass die Unterseite (1) des Kissens auch im aufgeblasenen Zustand im wesentlichen eben bleibt.

Gemäß einer besonders bevorzugten Ausführungsform der vorliegenden Erfindung weist das Kissen zusätzlich eine sich zwischen Unterseite und Oberseite befindliche Zwischenfolie (5) auf, deren Ränder mit den Rändern der Oberseite direkt verbunden sind, und über einen, über den gesamten Rand des Kissens verlaufenden Quersteg (6) mit der Unterseite verbunden ist.

Dieses Kissen mit Zwischenfolie (5) kann zusätzlich mindestens einen Mittelsteg (4) aufweisen, der in Längsrichtung des Kissens verläuft und mit der Zwischenfolie und der Unterseite verbunden ist. Je nach Elastizität des verwendeten Folienmaterials kann es aber auch notwendig sein, mehrere Mittelstege zu verwenden, wobei die Abstände zwischen den einzelnen Mittelstegen nicht äquidistant sein müssen.
Hierdurch wird auf besonders effektive Weise eine zu starke Krümmung der Unterseite (1) des Kissens vermieden, um die orthopädisch so wichtige Stützfunktion der Mulde (3) des Kissens zu gewährleisten.

Sofern das Kissen einen Quersteg und/oder mindestens einen Mittelsteg aufweist, können die durch Quersteg und oder Mittelsteg gebildeten Kammern durch im Inneren des Kissens befindliche Öffnungen miteinander verbunden sein.
Hierdurch ist es möglich, dass ein solches Kissen nur ein Lufteinlassventil aufweist, was seine Handhabung besonders vereinfacht.

In einer weiteren erfindungsgemäßen Ausführungsform weist das Kissen zusätzlich eine Fixiervorrichtung (7) zur Umspannung einer Sitz- und/oder Liegevorrichtung auf. Diese Fixiervorrichtung dient zur Befestigung des Kissens mit der unter dem Kissen befindlichen Sitz- und/oder Liegevorrichtung, beispielsweise einer Massagebank.
Die Fixiervorrichtung (7) kann einen elastischen Bereich und eine frei lösbare Verspannungsvorrichtung (8) aufweisen, wie beispielsweise in Figur III dargestellt.
Sofern das Kissen öfter von der Sitz- und/oder Liegevorrichtung entfernt bzw. angebracht werden muss, handelt es sich bei der Verspannungsvorrichtung (8) vorzugsweise um einen Klettverschluss.
Darüber hinaus kann auf diese Weise ein Frotteetuch zwischen dem Kissen und der Sitz- und/oder Liegevorrichtung fixiert werden, was insbesondere aus hygienischen Gesichtspunkten von besonderer Bedeutung ist.

Entsprechend einer ebenfalls besonders bevorzugten Ausführungsform weist das Kissen zwei, in Längsrichtung nebeneinander angeordnete Mulden (3) und (3a) auf. Ein solches Kissen ist beispielsweise in Figur III dargestellt und wird insbesondere zur orthopädischen Behandlung von Erkrankungen im Kniebereich verwendet.

In einer besonderen Ausführungsform der vorliegenden Erfindung weist das Kissen mindestens eine, zum Kisseninneren hin gerichtete Einbuchtung (9) auf, insbesondere kann es an beiden, die Breite des Kissens ausmachende äußere Seite Einbuchtungen (9) und (9a) haben.
Eine solche Ausführungsform wird immer dann gewählt, wenn die orthopädische Verwendung eines erfindungsgemäßen Kissens vornehmlich auf die Entlastung der entsprechenden Druckpunkte im Hals (Occiput und Schultergürtel) bzw. Knie (Wadenmuskulatur und ischiocruale Muskulatur) abzielt. Ferner können durch solche Mulden wirksam Sensibilitätsstörungen sowie Ausfallerscheinungen der Motorik im Arm- und Schulterbereich vermieden werden.

Bei dem erfindungsgemäßen Kissen sind die Folien insbesondere aus einem elastischen und/oder thermoplastischen Material, insbesondere aus Latex oder PVC.
Hierdurch wird eine optimale anatomische Anpassung an die entsprechenden Körperpartien gewährleistet.

Das Kissen kann des weiteren auf der Oberseite Anformungen und/oder Verstärkungen (10) im Bereich der Ausnehmungen zur Profilierung der Oberflächenkontur aufweisen.
Die Lage, Größe und Ausrichtung der Anformungen und/oder Verstärkungen richtet sich nach der gewünschten Vorformung.
Sofern das Kissen hauptsächlich eine seitliche Stützfunktion erfüllen soll, verlaufen die Anformungen und/oder Verstärkungen im wesentlichen in Längsrichtung des Kissens. Besonders bevorzugt handelt es sich bei den Anformungen und/oder Verstärkungen um Verschweissungen.

Des weiteren können die Folien des Kissen aus einem transparenten, insbesondere aus einem durchsichtigen Material, bestehen.
Ein solches Kissen kann dann auch im Inneren einen Werbeaufdruck (11) aufweisen, insbesondere befindet sich dieser Werbeaufdruck (11) auf der zum Kisseninneren zeigenden Seite der Unterseite oder auf der zur Kissenoberseite gerichteten Seite der Zwischenfolie (5).

So kann auf sehr effektvolle Weise ein durch die Kissenoberseite gut sichtbare Werbung aufgebracht werden.

Durch Wahl eines weniger elastischen Materials für die Kissenunterseite bzw. Zwischenfolie können überdies Verzerrungen des Werbeaufdrucks so gut wie ausgeschlossen werden.

Darüber hinaus kann das Kissen eine Breite zwischen 65 und 70 cm, eine Länge zwischen 18 und 20 cm und eine Höhe zwischen 6 und 8 cm aufweisen.
Diese Größe macht es universell verwendbar für die gebräuchlichsten orthopädischen Behandlungsmethoden.

Das zuvor beschriebene Kissen wird entsprechend einer weiteren Aufgabe der vorliegenden Erfindung als anatomisch vorgeformtes Kissen, insbesondere zur orthopädischen, präventiven und postoperativen Behandlung von Erkrankungen der Halswirbelsäule, Schulter und/oder des Kniegelenks verwendet.

Die folgenden Figuren I bis III dienen der Erläuterung der Erfindung.

Figur I zeigt die Aufsicht und Figur II den Querschnitt entlang der Breite eines erfindungsgemäßen Kissens mit einer Mulde (3), einer zwischen der Unterseite (1) und der Oberseite angeordneten Zwischenfolie (5), einen den äußeren Rand des Kissens bildenden Quersteg (6) und mehreren Mittelstegen zur Stabilisierung einer ebenen Unterseite.

Figur III zeigt den Querschnitt entlang der Breite eines erfindungsgemäßen Kissens mit zwei Mulden (3) und (3a) und ohne Zwischenfolie, dafür aber mit einer Fixiervorrichtung (7) mit Verspannungsvorrichtung (8).

In den Figuren werden folgende Bezugszeichen verwendet (in numerischer Reihenfolge):
(1) Unterseite
(2) und (2a) konvex gewölbte Oberseite
(3) und (3a) konkav verlaufende Mulde
(4) Mittelsteg
(5) Zwischenfolie
(6) Quersteg
(7) Fixiervorrichtung
(8) Verspannungsvorrichtung
(9) Einbuchtung
(10) Anformungen/Verstärkungen
(11) Werbeaufdruck

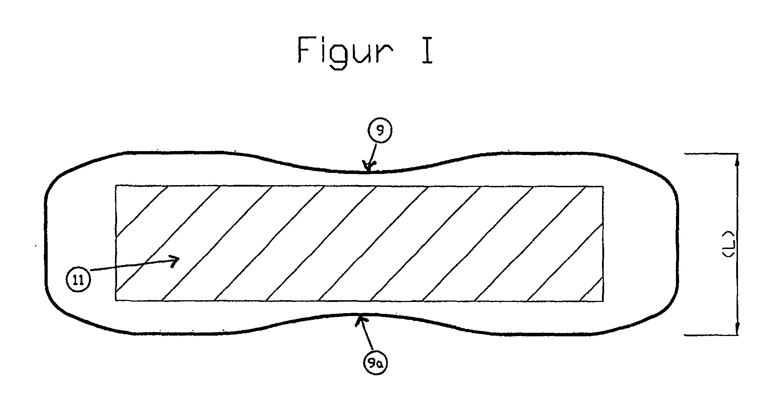

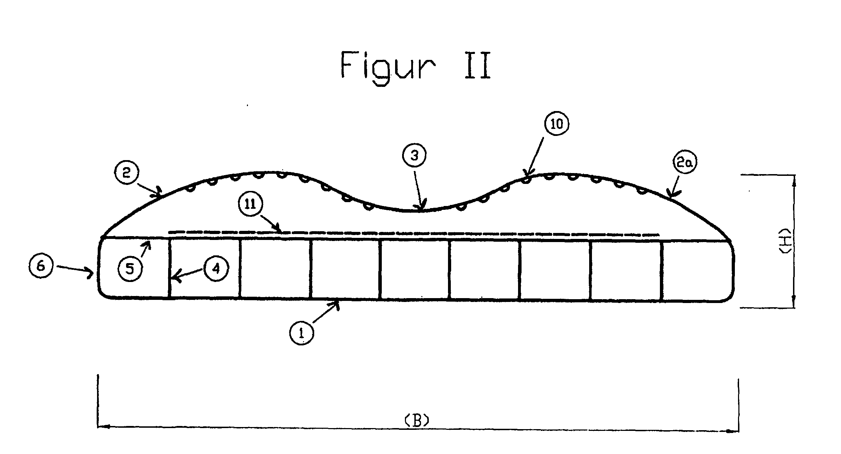

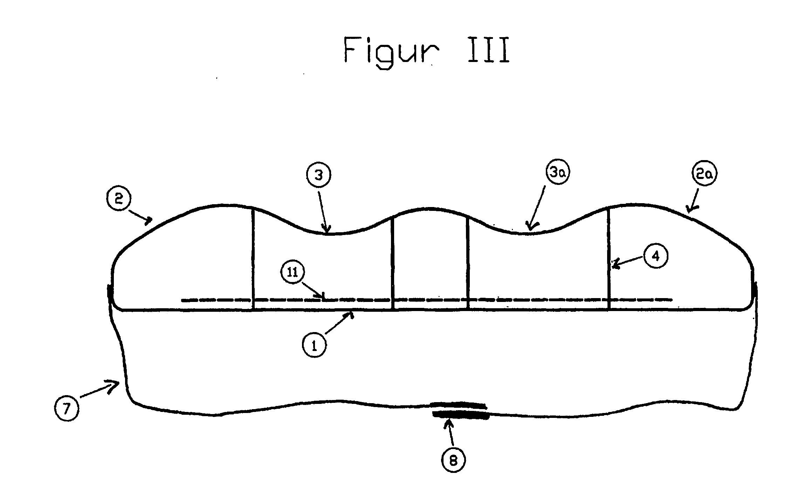

## Patentansprüche

1. Aufblasbares Kissen aus mindestens zwei miteinander verbundenen Folien, von denen die eine Folie die Unterseite des Kissens bildet und die andere Folie elastomere Eigenschaften aufweist und die Oberseite des Kissens bildet, im aufgeblasenen Zustand mit einer Breite (B) zwischen 50 und 80 cm, einer Länge (L) zwischen 10 und 30 cm, und einer Höhe (H) von maximal 10 cm, umfassend eine im wesentlichen ebene Unterseite (1), eine im Querschnitt der Längsrichtung betrachtet an beiden Randbereichen je eine konvex gewölbte Oberseite (2) und (2a) und mindestens eine, zwischen den Randbereichen in Richtung der Unterseite konkav verlaufende Mulde (3) der Oberseite.

2. Kissen nach Anspruch 1 **dadurch gekennzeichnet, dass** es zusätzlich mindestens einen Mittelsteg (4) aufweist, der in Längsrichtung des Kissens verläuft und mit der Oberseite und der Unterseite verbunden ist.

3. Kissen nach Anspruch 1, **dadurch gekennzeichnet, dass** es zusätzlich eine zwischen Unterseite und Oberseite befindliche Zwischenfolie (5) aufweist, deren Ränder mit den Rändern der Oberseite direkt verbunden sind, und die über einen, über den gesamten Rand des Kissens verlaufenden Quersteg (6) mit der Unterseite verbunden ist.

4. Kissen nach Anspruch 3, **dadurch gekennzeichnet, dass** es zusätzlich mindestens einen Mittelsteg (4) aufweist, der in Längsrichtung des Kissens verläuft und mit der Zwischenfolie und der Unterseite verbunden ist.

5. Kissen nach Anspruch 3 oder 4, **dadurch gekennzeichnet, dass** die durch Quersteg und/oder Mittelsteg gebildeten Kammern durch im Inneren des Kissens befindliche Öffnungen miteinander verbunden sind.

6. Kissen nach Anspruch 5 **dadurch gekennzeichnet, dass** es nur ein Lufteinlassventil aufweist.

7. Kissen nach einem der vorhergehenden Ansprüche, **dadurch gekennzeichnet, dass** es zusätzlich eine Fixiervorrichtung (7) zur Umspannung einer Sitz- und/oder Liegevorrichtung aufweist.

8. Kissen nach Anspruch 7, **dadurch gekennzeichnet, dass** die Fixiervorrichtung (7) einen elastischen Bereich und eine frei lösbare Verspannungsvorrichtung (8) aufweist.

9. Kissen nach Anspruch 8, **dadurch gekennzeichnet, dass** die Verspannungsvorrichtung (8) ein Klettverschluss ist.

10. Kissen nach einem der vorhergehenden Ansprüche, **dadurch gekennzeichnet, dass** es zwei, in Längsrichtung nebeneinander angeordnete Mulden (3) und (3a) aufweist.

11. Kissen nach einem der vorhergehenden Ansprüche, **dadurch gekennzeichnet, dass** es mindestens eine, zum Kisseninneren hin gerichtete Einbuchtung (9) aufweist.

12. Kissen nach Anspruch 11, **dadurch gekennzeichnet, dass** es an beiden, die Breite des Kissens ausmachende äußere Seite Einbuchtungen (9) und (9a) aufweist.

13. Kissen nach einem der vorhergehenden Ansprüche, **dadurch gekennzeichnet, dass** die Folien erhältlich sind aus einem elastischen und/oder thermoplastischen Material, insbesondere aus Latex oder PVC.

14. Kissen nach einem der vorhergehenden Ansprüche, **dadurch gekennzeichnet, dass** die Oberseite im Bereich der Ausnehmungen Anformungen und/oder Verstärkungen (10) zur Profilierung der Oberflächenkontur aufweist

15. Kissen nach Anspruch 14, **dadurch gekennzeichnet, dass** die Anformungen und/oder Verstärkungen (10) in Längsrichtung des Kissens verlaufen und insbesondere Verschweissungen sind.

16. Kissen nach einem der vorhergehenden Ansprüche, **dadurch gekennzeichnet, dass** die Folien aus einem transparenten, insbesondere aus einem durchsichtigen Material, bestehen.

17. Kissen nach Anspruch 16, **dadurch gekennzeichnet dass** die Unterseite oder die Zwischenfolie einen auf der Innenseite des Kissens befindlichen Werbeaufdruck (11) aufweist.

18. Kissen nach einem der vorhergehenden Ansprüche, **dadurch gekennzeichnet, dass** die Breite zwischen 65 und 70 cm, die Länge zwischen 18 und 20 cm und die Höhe zwischen 6 und 8 cm liegt.

19. Verwendung eines Kissens nach einem der vorhergehenden Ansprüche als anatomisch vorgeformtes Kissen, insbesondere zur orthopädischen, präventiven und postoperativen Behandlung von Erkrankungen der Halswirbelsäule, Schulter und/oder des Kniegelenks.

## Claims

1. Inflatable cushion comprising of at least two joint foils wherein one of the foils forms the underside of the cushion and the other foil having elastomaric properties forms the top face of the cushion being between 50 and 80 cm wide, between 10 and 30 cm long and 10 cm at max. high when inflated and comprising of an essentially flat underside (1) and in the cross view along the longitudinal axis on both edges convex arched top face (2) and (2a) and at least one in direction of the underside concave shaped trough (3) between the edges of the top face.

2. Cushion according to claim 1 **characterised in that** the cushion has additionally at least one centre bridge in the longitudinal direction being linked with the underside and the top face of the cushion.

3. Cushion according to claim 1 **characterised in that** the cushion has additionally an intermediate foil (5) between the underside and the top face which edges are directly linked with the edges of the top face and which is linked with the underside via cross-bar running along the edges of the cushion.

4. Cushion according to claim 3 **characterised in that** the cushion additionally comprises of at least one centre bridge (4) running in longitudinal direction and being linked with the under side via an intermediate foil.

5. Cushion according to claims 3 or 4 **characterised in that** the chambers built by the cross-bar and/or the centre bridge are linked by internal openings with each other.

6. Cushion according to claim 5 **characterised in that** it comprises only one intake valve.

7. Cushion according to one of the preceding claims **characterised in that** the cushion additionally comprises a fixation (7) to surround a seat or bed.

8. Cushion according to claim 7 **characterised in that** the fixation (7) comprises an elastic area and a fastening (8) being freely loosenable.

9. Cushion according to claim 8 **characterised in that** the fastening (8) is a Velcro fastener.

10. Cushion according to one of the preceding claims **characterised in that** the cushion comprises two troughs running in longitudinal direction (3) and (3a).

11. Cushion according to one of the preceding claims **characterised in that** the cushion comprises at least one dent (9) directed to the internal of the cushion.

12. Cushion according to claim 11 **characterised in that** the cushion comprises of dents (9) located on both sides of the cushion which form the width of the cushion.

13. Cushion according to one of the preceding claims **characterised in that** the foils are made of an elastic and/or thermoplastic material, preferably of latex or PVC.

14. Cushion according to one of the preceding claims **characterised in that** the top face having reinforcements and/or rims (10) in the area of the omissions to profile the surface contour.

15. Cushion according to claim 14 **characterised in that** the rimes and/or reinforcements (10) run in longitudinal direction of the cushion and are preferably jig welded.

16. Cushion according to one of the preceding claims **characterised in that** the foils consist of a transparent preferably of a lucid material.

17. Cushion according to claim 16 **characterised in that** underside or the intermediate foil comprise an advertising print (11) on the inside of the cushion.

18. Cushion according to one of the preceding claims **characterised in that** the cushion is between 65 and 70 cm wide, between 18 and 20 cm long, and between 6 and 8 cm high.

19. Use of the cushion according to one of the preceding claims as an anatomically formed cushion especially for orthopaedic, preventive and post operative treatment of spine diseases and/or of diseases of the shoulder and/or of the knee.

## Revendications

1. Coussin gonflable constitué d'au moins deux feuilles reliées entre elles dont l'une forme la partie inférieure du coussin et l'autre présente des propriétés élastomères et forme la partie supérieure du coussin, ayant à l'état gonflé une largeur (B) comprise entre 50 et 80 cm, une longueur (L) comprise entre 10 et 30 cm et une hauteur (H) de 10 cm maximum, comprenant une partie inférieure (1) essentiellement plate, une partie supérieure qui, vue de profil, a dans le sens de la longueur respectivement sur les deux bords une courbure convexe (2) et (2a) et, entre les deux bords, au moins une excavation concave (3) vers la partie inférieure.

2. Coussin selon la revendication 1, **caractérisé en ce qu'**il présente en plus au moins une bordure médiane (4) dans le sens de la longueur du coussin et reliée avec la partie supérieure et avec la partie inférieure.

3. Coussin selon la revendication 1, **caractérisé en ce qu'**il présente en plus une feuille intermédiaire (5) située entre la partie inférieure et la partie supérieure dont les bords sont directement reliés avec les bords de la partie supérieure et qui est reliée avec la partie inférieure par une bordure transversale (6) située sur tout le bord du coussin.

4. Coussin selon la revendication 3, **caractérisé en ce qu'**il présente en plus au moins une bordure médiane (4) dans le sens de la longueur du coussin reliée avec la partie supérieure et avec la partie inférieure.

5. Coussin selon l'une des revendications 3 ou 4, **caractérisé en ce que** les chambres formées par la bordure transversale et/ou médiane sont reliées entre elles par des ouvertures se trouvant à l'intérieur du coussin.

6. Coussin selon la revendication 5, **caractérisé en ce qu'**il ne présente qu'une seule soupage d'entrée d'air.

7. Coussin selon l'une des revendications précédentes, **caractérisé en ce qu'**il présente en plus un dispositif de fixation (7) pour mettre autour d'un siège ou d'une couchette.

8. Coussin selon la revendication 7, **caractérisé en ce que** le dispositif de fixation (7) présente une partie élastique et un dispositif de consolidation (8) facilement détachable.

9. Coussin selon la revendication 8, **caractérisé en ce que** le dispositif de consolidation (8) est une bande velcro.

10. Coussin selon l'une des revendications précédentes, **caractérisé en ce qu'**il présente deux excavations (3) et (3a) situées l'une à côté de l'autre dans le sens de la longueur.

11. Coussin selon l'une des revendications précédentes, **caractérisé en ce qu'**il présente au moins un creux (9) vers l'intérieur du coussin.

12. Coussin selon la revendication 11, **caractérisé en ce qu'**il présente des creux (9) et (9a) sur les deux côtés extérieurs formant la largeur du coussin.

13. Coussin selon l'une des revendications précédentes, **caractérisé en ce que** les feuilles sont susceptibles d'être obtenues à partir d'une matière élastique et/ou thermoplastique, notamment de latex ou de PVC.

14. Coussin selon l'une des revendications précédentes, **caractérisé en ce que** la partie supérieure présente, à l'endroit des creux, des modelages et/ou des renforcements (10) pour profiler les contours de la surface.

15. Coussin selon la revendication 15, **caractérisé en ce que** les modelages et/ou renforcements (10) sont situés dans le sens de la longueur du coussin et sont notamment soudés.

16. Coussin selon l'une des revendications précédentes, **caractérisé en ce que** les feuilles sont composées d'une matière diaphane et en particulier transparente.

17. Coussin selon la revendication 16, **caractérisé en ce que** la partie inférieure ou la feuille intermédiaire présente une impression publicitaire (11) se trouvant sur le côté intérieur du coussin.

18. Coussin selon l'une des revendications précédentes, **caractérisé en ce que** la largeur est comprise entre 65 et 70 cm, la longueur entre 18 et 20 cm et la hauteur entre 6 et 8 cm.

19. Utilisation d'un coussin selon l'une des revendications précédentes en tant que coussin de forme anatomique, notamment pour le traitement orthopédique, préventif et post-opératoire des maladies des vertèbres cervicales, des épaules et/ou des articulations des genoux.
